# EUROPEAN PATENT SPECIFICATION

(11) **EP 4 287 944 B1**
(45) Date of publication and mention of the grant of the patent: **04.06.2025**
(21) Application number: 21704204.3
(22) Date of filing: 03.02.2021
(51) Int. Cl.: A61B 5/1455, A61B 5/0205, A61B 5/021, A61B 5/024, A61B 5/01, A61B 5/08, A61B 5/00, G16H 50/30

(54) **A WEARABLE SYSTEM COMPRISING AN EAR-WORN DEVICE FOR PROVIDING AN EARLY WARNING SCORE**
TRAGBARES SYSTEM MIT EINER AM OHR GETRAGENEN VORRICHTUNG ZUR BEREITSTELLUNG EINER FRÜHWARNBEWERTUNG
SYSTÈME POUVANT ÊTRE PORTÉ COMPORTANT UN DISPOSITIF PORTÉ SUR L'OREILLE PERMETTANT DE FOURNIR UN SCORE D'ALERTE PRÉCOCE

(43) Date of publication of application: 13.12.2023
(73) Proprietor: Widex A/S, 3540 Lynge (DK)
(72) Inventor: ANDERSEN, Svend Vitting, 3540 Lynge (DK); NIELSEN, Kim Hjortgaard, 3540 Lynge (DK)
(74) Representative: Widex A/S
(86) International application number: PCT/EP2021/052520
(87) International publication number: WO 2022/167065

(56) References cited:
- WO-A1-2018/085563
- US-A1- 2018 132 794
- US-A1- 2019 212 198
- ANZANPOUR ARMAN ARMANZ@UTU FI ET AL: "Edge-Assisted Control for Healthcare Internet of Things", ACM TRANSACTIONS ON INTERNET OF THINGS, ACMPUB27, NEW YORK, NY, USA, vol. 2, no. 1, 19 October 2020 (2020-10-19), pages 1 - 21, XP058492033, DOI: 10.1145/3407091

## Description

The invention is related to a wearable system comprising an ear-worn device, wherein by means of a number of sensors of the ear-worn device, biometric quantities of a user of the wearable system are assessed out of: a heart rate, an oxygen saturation of the blood, a blood pressure and a respiratory rate. Body-worn systems, so called "wearables", increasingly come equipped with functionalities for monitoring, in the broadest sense, biological activities of a wearer's body, such as heart rate or blood pressure. In particular, such systems gained popularity among the sports-oriented public, as these systems may support a routine of workout or running by adding a monitoring of the body's response to the physical exercise, on top of the functionalities related more to workout in its strict sense, such as a chronometer, step counting and the like. These wearable systems comprise at least one body-worn unit that contains respective sensors for measuring the biological activities mentioned above, and which is disposed at a location on the wearer's body suitable for said measurements.

Such systems also may comprise one or two ear-worn devices. In many implementations, two ear-worn devices of the wearable system, at least in an additional function, serve as headphones for playing music to the wearer's earing during the workout routine. Furthermore, an ear-worn device may comprise a microphone (or a similar electroacoustic input transducer) for receiving voice commands from the wearer in order to control the systems functions. US 2019/212198 A1 discloses such an ear-worn device.

As already mentioned, wearable systems of the described type are widely used in relation with physical activity.

However, a monitoring of critical health conditions of a person, in particular of a medically vulnerable person, would be desirable, in the sense that an automatized routine may output an alert in a suitable way when a state of health of a system's wearer, i.e., the user, reaches a critical condition, such that medical staff can get informed and attend the user personally.

WO 2018/085563 A1 discloses an early warning scoring system and method. The system comprises a computing device, a plurality of sensors for acquiring physiological signals from a patient, wherein the sensors are functionally connected to the computing device, and at least one alarm adapted to output an alert upon an early warning score (EWS) exceeding a predetermined level. The computing device receives the physiological signals from the sensors, analyzes the physiological signals, and based on the analyzed signals, calculates the early warning score, and compares to the early waring score to predetermined limits and, if the score is outside the limits, triggers an alarm or actuates or modifies a treatment or medical intervention. The sensors obtain patient data relating to at least one of minute ventilation, tidal volume, respiratory rate, oxygen saturation, temperature, blood pressure, pulse or heart rate, blood oxygen levels, and brain activity. The computing device further obtains patient data comprising alertness, voice, pain, and unresponsiveness (AVPU) of the patient, and the EWS calculation includes the patient's AVPU data.

It is therefore an object of the present invention to present a wearable system such that in case of a medically critical condition of the system's user, an alert can be output in a suitable way. It is furthermore the object of the invention to present a wearable system capable of monitoring its user's state of health and generating such an alert if estimated necessary.

According to an aspect of the disclosure, the object is achieved by a method for providing an early warning score (EWS) by means of a wearable system comprising an ear-worn device, wherein by means of a number of sensors of the ear-worn device, at least three biometric quantities of a user of the wearable system are assessed out of: a heart rate, an oxygen saturation of the blood, a blood pressure and a respiratory rate, wherein each to of the at least three biometric quantities, a preferably non-negative criticality value is assigned, and wherein from said criticality values, an EWS is derived. Embodiments of particular advantage, which may be inventive in their own right, are outlined in the depending claims and in the following description.

According to the invention, the object is achieved by a wearable system comprising an ear-worn device, wherein the ear-worn device comprises a number of sensors, each of which configured to generate a respective sensor signal when the wearable system is worn by a user of the wearable system, each sensor signal being representative of at least one vital function activity of said user, wherein the wearable system is configured to derive at least three biomedical quantities of the user out of the following from the sensor signals: a heart rate, an oxygen saturation of the blood, a blood pressure and a respiratory rate, and to derive an EWS from said biomedical quantities.

The wearable system according to the invention shares the advantages of the method according to the disclosure. Particular assets of the method and of its embodiments may be transferred, in an analogous way, to the wearable system and its embodiments, and vice versa.

The EWS is a standardized score based on a person's vital function activity and vital signs, in particular, on the respiratory rate, the oxygen saturation in the blood, the blood pressure, the body temperature, the heart rate and a level of consciousness. Biometric quantities associated to said vital function activities and signs are mapped onto discrete, non-negative integer values, and a sum of these values gives one single number allowing for a quick assessment of possible urgent health problems of a person which may need medical attention. In this respect, the present invention goes one step further and defines the EWS as a more general concept: in order to provide such a single number score by means of sensors that may all be included in an ear-worn device, the biometric quantities on which said single number score shall be based, may be limited with respect to the conventional definition of the EWS as given above. Thus, it shall be considered a sufficiently close approximation to derive this "general" EWS based on at least three biometric quantities out of the heart rate, the oxygen saturation, the blood pressure and the respiratory rate.

These biometric quantities are assessed by means of a number of sensors located, as mentioned above, in the ear-worn device, such that from the sensor signals of said sensors (or from a single sensor signal of a single sensor, given the case) the biometric quantities are representable in floating point or integer numbers. Each of these numbers is mapped by a predefined, tabulated rule onto a set of criticality values, i.e., to each of the biometric quantities, one such criticality value from the predefined set is assigned.

Preferably, the criticality values are given by non-negative integer values, and most preferably, a criticality value of 0 indicates a normal vital function activity for the corresponding biometric quantity, while extremely significant deviations from normal vital function activity shall be represented by a criticality value of 3. This, however, is only a preferred choice, and a different scaling may be defined. Regardless of the particular scaling used, in order to make the different biometric quantities comparable to each other such that they may all contribute to the EWS, it is of advantage to divide the full range of a biometric quantity's values into several intervals, said intervals being mapped onto the respective criticality values.

From these criticality values, the EWS according to the invention shall be derived as a monotonous function. Preferably, the monotonous function of the criticality values is given simply by a sum, however, other functions, e.g., linear functions with different weights for different biometric quantities or with a non-linear (yet monotonous) scaling are also possible.

The notion of a wearable system shall in particular comprise any single device to be worn by a user somewhere on his body, and any system of devices connectable (in particular, wirelessly) with each other, wherein at least one of the devices is worn by a user on his body hearing normal operation of the system. The notion of an ear-worn device shall in particular comprise any device which for its intended use, and during its normal operation shall be worn by its user on or at or in one of his years, e.g., a hearing aid, a headset, and earplug and the like. In the present case, if the wearable system consists only of one single device, said device is given by the ear-worn device.

The invention allows for providing an EWS - in its more general form - in order to obtain a single number value for a quick assessment of possible urgent health problems of a user of the wearable system. This EWS simply can be monitored on a long-term basis (e.g., for identifying an evolution of a person's conditions), or may be used for an immediate action.

In this respect, preferably, the EWS is compared to a first threshold, wherein an alert signal is transmitted to a remote medical monitoring server and/or to a medical service person, such as a general practitioner or a care person, when the EWS exceeds the first threshold. This may be particularly useful for older persons or other persons who have general health problems which might cause sudden deteriorations of their vital function activities and vital signs.

In an embodiment, the alert signal is transmitted from the ear-worn device to the remote medical monitoring server and/or to the medical service person, respectively, via a low-power wide-area network (LPWAN). In particular, a commercial network system, such as LoRa or Sigfox, may be used to this end. An LPWAN has the advantage of a low power consumption for the message transmission over distances of various km, such that the communication link for transmission may be established from the ear-worn device by a corresponding low-power sending device disposed therein.

Advantageously, as a further biometric quantity of the user, a body temperature is assessed by means of a temperature sensor of the wearable system, and a respective criticality value is assigned to the assessed body temperature, wherein the EWS is further derived from said criticality value corresponding to the body temperature. The conventional definition of the EWS of a person is based also on the body temperature of the person. Preferably, said temperature sensor is arranged in the ear-worn device. In particular, the notion of a temperature sensor shall comprise any type of sensor arranged and configured to measure a person's body temperature.

According to the invention, a level of consciousness of the user is assessed as a further biometric quantity, and a respective criticality value is assigned to the assessed level of consciousness, wherein the EWS is further derived from said criticality value corresponding to the level of consciousness. The conventional definition of the EWS of a person is also based on the person's level of consciousness, typically given in the AVPU scale ("alert, verbal, pain, unresponsive", wherein verbal and pain indicates that a person shows a perceivable reaction to a verbal or pain stimulus). For a scaling of criticality values by using only non-negative integer values, the levels of consciousness A, V, P, and U may be directly mapped onto the integers from 0 to 3 in ascending order.

According to the invention, the wearable system is configured to derive a preliminary warning score (PWS) from criticality values corresponding to at least some biometric quantities other than the level of consciousness (i.e., from criticality values corresponding to at least some out of heart rate, oxygen saturation, blood pressure, respiratory rate and body temperature), wherein the level of consciousness is assessed when the preliminary warning score exceeds a critical preliminary value, and wherein the EWS is derived based on the PWS and the level of consciousness. Since assessing a level of consciousness using the wearable system, and particularly using the ear-worn device, may be involved, this operation may be restricted to the case when the PWS indicates, by exceeding the critical preliminary value, that the EWS might attain a significant value.

In an embodiment, an environment sound is picked up and converted into an audio input signal by means of an electroacoustic input transducer of the ear-worn device, wherein by means of a sound pattern recognition containing an own voice detection, an utterance of the user is recognized in the audio input signal, and wherein the level of consciousness of the user is assessed upon the detection of the utterance. The utterance, e.g., may be recognized as a pain utterance, and it can then be deduced that the user's level of consciousness is at least "P" in the AVPU scale.

Preferably, the level of consciousness is assessed by means of an electroencephalographic (EEG) sensor of the ear-worn device. Normally, an EEG sensor comprises at least two different electrodes in order to determine a potential difference between the two electrodes. For a detailed measurement of neural activities, the electrodes are typically distributed on different measurement points of a person's the head, preferably enclosing as much of the person's brain as possible. However, for assessing a level of consciousness of an ear-worn device's user in the AVPU scale, it may be sufficient to evaluate the user's neural reaction only at measurement points accessible via the ear-worn device. Even though such an EEG may not resolve the full detailed neural activity of the user, the mere existence of a neural reaction to an acoustic or pain stimulus may still be visible from the EEG measurement performed by the ear-worn device only.

It results to be of further advantage to present at least one acoustic stimulus to a hearing of the user by means of an output speaker of the ear-worn device, wherein the level of consciousness is assessed by means of measuring a reaction of the user's brain to the acoustic stimulus, in particular, a neural reaction via the EEG, but also an answer via a microphone of the wearable system. Said acoustic stimulus can be given by a signal tone or by an automatic dialogue protocol or even by a phone call with some special medical call center. The level of consciousness, then, may be assessed as at least "V" if the user reacts in some way - either by responding, or by his/her neural activity - to the acoustic stimulus. By means of an automatic dialogue protocol, even a fully alert level ("A") may be distinguished from merely reacting to verbal stimuli.

The level of consciousness may also be assessed in terms of the so-called "Glasgow Coma Scale" (GCS). This scale is used to measure a person's level of consciousness in particular after a brain injury. For the whole GCS, a person's ability to perform eye movements, speech and body movements are separately assessed, and the ability in each of the three mentioned categories is classified, normally with an integer from 1 to 6, where 1 represents the disability to move eyes, to speak or to move any parts of the body, respectively. The sum of the three respective integers assigned to eye movement, speaking ability and body movement then yields the GCS.

In the framework of the present invention, the wearers speaking ability may be assessed by recognizing utterances of the wearer by means of the electroacoustic input transducer of the ear-worn device and sound pattern recognition as described above, and the level of speaking ability may be classified according to the respective scale as used for the GCS. In particular, an automatic conversation protocol may be implemented via the output speaker and the electric input transducer of the ear-worn device, such that an output sound with a message to the wearer is presented to his/her hearing and an answer, or more generally, the speech reaction is assessed by means of the electroacoustic input transducer. From the answer, the next message according to the protocol can be presented to the wearer's hearing until the degree of speech activity may be assessed in terms of the GCS.

Furthermore, the eye movement may be assessed by using the α band component of the EEG signal (at approx. 8-10 Hz). It has been shown that the EEG signal has a distinguishably higher amplitude in this band when a person has his/her eyes closed. Thus, an assessment whether eyes are open spontaneously, or whether they open to a normal voice command, to a pain stimulus (both of which preferably to be emitted by the output speaker as a voice command or a pain stimulus contained in the output sound, respectively), or not at all, is possible by means of the EEG sensor of the ear-worn device.

Finally, the body movement of the wearer may be assessed, at least approximately and within the scope of the GCS, by at least one accelerometer disposed in the wearable system. Preferably, the accelerometer is disposed in the ear-worn device of the wearable system. However, if the wearable system comprises one or more further body-worn devices such as a smart-watch or the like, an accelerometer may be disposed there, as well (either as an alternative to the ear-worn device or additionally).

The assessed integer values to the eye and/or body movement and/or to speech activity may be combined to the full GCS or to a restricted version (in case that not all three components are available), and the GCS may be mapped in a proper way - in particular, by respective scaling - onto a respective criticality value to be included in the EWS.

It is of further advantage to estimate an approximation for the level of consciousness, wherein upon estimating a critical level of consciousness, a communication link between the wearable system and a medical call center is established for communication between a medical service person of the medical call center and the user in order to assess the user's level of consciousness. This way, the medical service person can assess in detail the level of consciousness of the user, in case that there are doubts (in particular, whether the user is alert or merely reacts, i.e., A vs. V). A medical call center may be any kind of communication call center suitably configured and arranged for this purpose. The medical service person in this case may be given by any staff of the medical call center.

Preferably, the PWS is compared to a second threshold, and, when the preliminary warning score exceeds the second threshold, an alert information is presented to the user by means of the wearable system, e.g., as an audible alert information from the ear-worn device or an optical alert information from a screen of an auxiliary device of the wearable system. Preferably, the second threshold is below the first threshold for the EWS, such that the user gets informed when a deterioration of his medical state is in the beginning, allowing him to act accordingly, e.g., by contacting his/her general practitioner on his/her own, or by simply not getting exposed to situations possibly dangerous to his/her conditions. In particular, also the full EWS instead of the PWS may be used for said comparison with the second threshold.

Conveniently, the heart rate and/or the oxygen saturation and/or the blood pressure and/or the respiratory rate are assessed by means of a photoplethysmographic (PPG) sensor of the ear-worn device, and/or the respiratory rate is assessed by means of an or the audio input signal generated from an or the electroacoustic input transducer of the ear-worn device. Thus, the biometric quantities are directly available from the ear-worn device. It has already been demonstrated that a PPG sensor in ear-worn devices can measure heart rate and blood pressure. Respiratory rate can be measured by PPG using filtering techniques of the measured signal. Similarly, there exist advanced time analysis methods to extract the oxygen saturation from a PPG signal. Typical alternatives to heart rate and blood pressure measurements are chest straps and arm cuffs.

In an embodiment of the wearable system, the ear-worn device is given by a hearing device, in particular by a hearing aid. A hearing device shall be in particular understood as any device to be worn at an ear by a user, configured to provide a sound signal to the user's hearing at said ear. A hearing aid, in particular, shall be understood as a hearing device designed and configured to correct for a hearing impairment of its user. Hearing devices, on the one hand, are getting more distribution into the daily life, and are increasingly worn by persons, such that equipping a hearing device with the means to provide an EWS is an additional advantage.

On the other hand, older people or other persons with possible health problem, often are wearing hearing aids anyway due to some degree of a hearing impairment, such that an EWS for these people may be obtained without the need for additional, dedicated devices the user might lose, forget to wear, or get uncomfortable with.

Preferably, a first ear-worn device and a second ear-worn device configured to be worn by the user at different ears, wherein the first ear-worn device comprises at least first sensor of the number of sensors, and the second ear-worn device comprises at least a second sensor of the number of sensors measuring a different biometrical quantity than the first sensor. This means that some biometrical quantities are assessable only from sensor signals that are generated only in one of the ear-worn devices, while in particular, the ear-worn devices are hearing aids of a binaural hearing aid.

The attributes and properties as well as the advantages of the invention which have been described above are now illustrated with help of drawings of embodiment examples. In detail,
- figure 1: shows a schematic block diagram of a hearing device configured to assess biometric quantities for inferring an EWS of the hearing device's wearer,
- figure 2: shows a schematic block diagram of a process for deriving an EWS from the biometric quantities as assessed according to figure 1, and
- figure 3: shows a schematic block diagram of a binaural hearing aid configured to assess biometric quantities for inferring an EWS.

Parts and variables corresponding to one another are provided with the same reference numerals in each case of occurrence for all figures.

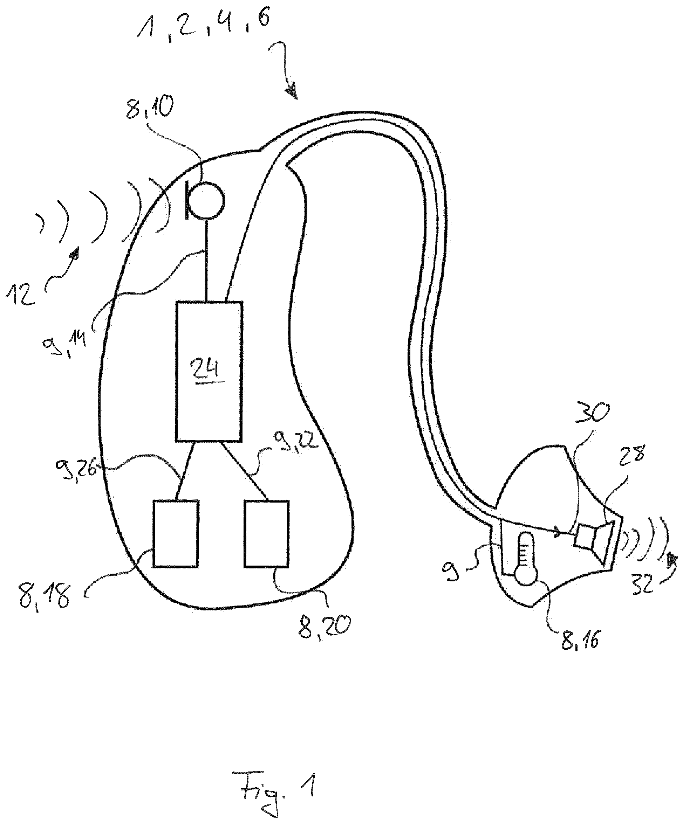

In figure 1, a schematic block diagram shows a wearable system 1, comprising an ear-worn device 2, by means of which an EWS of a user (not displayed) of the wearable system 1 may be provided. In the present embodiment, the wearable system 1 consists solely of the ear-worn device 2, which is given by a hearing device 4, in particular by a hearing aid 6 configured to support the user by correcting for a hearing loss. In figure 1, the hearing device 4 is schematically shown as a BTE-hearing aid ("behind the ear"), while the explanations and considerations are valid for any other type of hearing aid (or, in a broader sense, for any other type of hearing device) capable of enclosing the respective components such as sensors. The hearing device 4 comprises a number of sensors 8, all of which are configured to generate respective sensor signals 9, by means of which in a way yet to be described, vital function activities and vital signs of the user may be assessed or estimated. The locations of the sensors 8 with respect to the hearing device 4, as depicted in figure 1, are chosen for drawing purposes and are not necessarily to scale.

As one of the sensors 8, the hearing device for comprises at least one electroacoustic input transducer 10 (e.g., given by a microphone) configured to convert an environment sound 12 into an electric audio input signal 14. As a further sensor 8, the hearing device for comprises a temperature sensor 16 configured to measure a body temperature of the user. The temperature sensor 16 may be given by an optical sensor. As yet another sensor 8, the hearing device 4 comprises a PPG sensor 18. In general, a PPG sensor is an optical sensor measuring changes in a volume distribution of the human blood, and thus, also in its flow by measuring an oxygen saturation of the blood via measuring the transmission or reflection of a diode-emitted light in a specific region of human tissue. Furthermore, as yet another sensor 8, the hearing device 4 comprises an EEG sensor 20 configured to measure a neural activity of the user, thereby generating a respective EEG signal 22 as one of the sensor signals 9. The EEG sensor 20 may comprise several different electrodes (e.g., two electrodes; not shown in detail in figure 1) in order to measure said neural activity, e.g., as a difference of the electric potentials at the respective electrodes.

The sensors 8 are connected to a control unit 24 of the hearing device 4 comprising at least one signal processor for carrying out signal processing and control operations, such that the sensor signals 9 generated by the respective sensors 8 can be processed by said control unit 24 in order to assess said vital function activities of the user.

The sensor signals 9 are generated by the sensors 8 of the hearing device 4 in order to assess respective biometric quantities corresponding to a vital function activity of the user, from which the EWS shall be derived in a way yet to be described. To this end, as such biometric quantities of the user, a heart rate, and oxygen saturation of the blood and a blood pressure are directly derived from a PPG signal 26 generated by the PPG sensor 18. Today, the classical methods in the medical environment for measuring these quantities are normally implemented via chest straps, arm cuffs, clip-on finger or clip-on ear-flip devices and thermometers.

As further biometric quantities, a respiratory rate and a body temperature of the user are assessed. The respiratory rate may be inferred from the audio input signal 14 (under the assumption that the environment sound 12 contains an audible portion of the user's breathing activity), e.g., by a correlation measurement on said audio input signal 14, or may be inferred from amplitude variations of the PPG signal 26 in the order of magnitude of seconds, or from a combination of both. The body temperature of the user is directly measured by the temperature sensor 16.

In order to derive the standard EWS, apart from the heart rate, blood oxygen saturation, blood pressure, respiratory rate and body temperature, also an information of the wearer's level of consciousness is required. This level of consciousness is typically given by a value of the AVPU scale. One possibility to assess the level of consciousness of the user of the wearable system 1 is giving an acoustic stimulus to the user's hearing of the ear at which the ear-worn device 2 is worn, and register any type of verbal reaction by means of the electroacoustic input transducer 10 as corresponding sensor 8 (and by an appropriate processing of the audio input signal 14 with respect to the user's own voice and possible information about his level of consciousness contained therein), and any neural reaction by means of the EEG signal 22, and in particular its possible correlations to said acoustic stimulus. Such an acoustic stimulus may be presented to the hearing of the user by an output speaker 28, which is configured to convert an output signal 30 of the control unit 24 into an output sound signal 32 to be presented to the user's hearing. In order to assess the user's level of consciousness in terms of the AVPU scale, an acoustic stimulus in form of a simple question or a short question protocol, possibly triggered by an event yet to be described, is included in the output signal 30, such that the user may respond to the question or the question protocol.

A fully alert state (in the AVPU scale: "A") may be recognized by the user's verbal reactions to an automatized short question protocol designed in a way such that a full answering of all questions by the user ensures his full alert value of consciousness. As an alternative, or additionally, the user's neural activity while answering verbally may be measured via the EEG signal 22 in order to ensure a fully alert state. Any whatsoever reaction, be it verbal or only neural activities as derived from the EEG signal 26, to the question protocol that does not qualify for the fully alert level (in case of verbal reactions) may then be assessed as responsiveness on the verbal level (in the AVPU scale: "V").

If neither a verbal reaction of the user to the acoustic stimulus included in the output sound signal 32 can be extracted from the audio input signal 14, nor any neural reaction from the EEG signal 22, this indicates that the user may at best react to a small pain stimulus. In this case, one may either take a conservative stance and set the absence of any reaction to the acoustic stimulus as a total unresponsiveness (in the AVPU scale: "U"), as reactions to small pain stimuli may be difficult to measure. This may, at worst, overestimate the severity of the user's total state and, thus, might lead to a slightly too high EWS. As an alternative, one may take a short acoustic stimulus with an amplitude and frequency spectrum that are capable of causing a pain stimulus to the wearer (possibly, by taking into account the individual threshold of pain of the user which may be measured during the fitting of the hearing aid), such that a neural reaction - measured by means of the EEG signal 26 - to said pain stimulus contained in the output sound signal 32 may be used to infer that the user at least still is responsive to pain (in the AVPU scale: "P"). The total absence of any reactions to both the verbal and the pain stimuli then renders the level of consciousness as unresponsive (in the AVPU scale: "U"). Preferably, the EEG signal 26 is monitored in a way to ensure that the EEG sensor 20 is working properly and an absence of any measurable reaction in the EEG signal 26 to any verbal or pain stimuli is indeed due to the user being unresponsive (and not to any technical problem related to the EEG sensor 20). Likewise, the audio input signal 14 may be monitored in a similar way.

In an embodiment not shown in Figure 1, the level of consciousness may also be assessed according to the GCS, in particular by means of the output speaker 28, the electroacoustic input transducer 10, and the EEG sensor 20, and possibly an accelerometer (not shown) of the wearable system 1. An automatic conversation protocol may be implemented via the output speaker 28 and the electroacoustic input transducer 10 in order to assess the speaking ability of the wearer. Acoustic stimuli for opening the eyes as well as short pain stimuli may be emitted via the output speaker 28 in order to assess the eye movement from the EEG signal 26, and possibly to measure a body movement via said accelerometer.

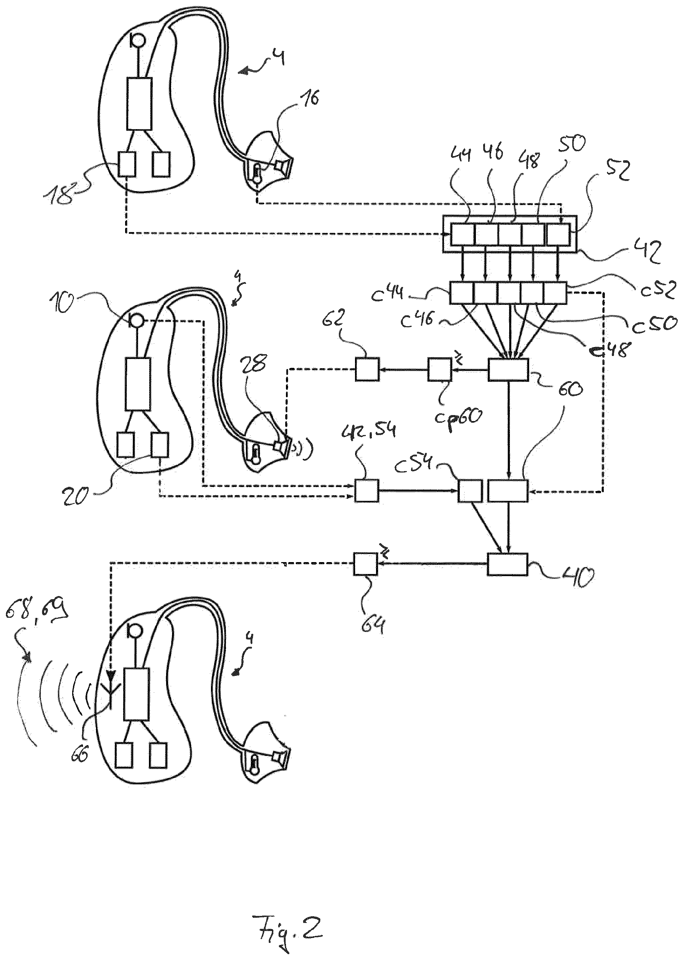

Figure 2 shows a schematic block diagram of a method for deriving an EWS 40 from biometric quantities 42 of the user which may be assessed as described according to figure 1.

By means of the PPG sensor 18 of the hearing device 4, the following biometric quantities 42 of the user are assessed in the way described above: a heart rate 44, an oxygen saturation 46 of the blood, a blood pressure 48 (preferably, a systolic blood pressure) and a respiratory rate 50 (for the sake of simplicity, in the embodiment shown in figure 2, the respiratory rate 50 is derived only from the PPG measurement). As a further biometric quantity 42 of the user, a body temperature 52 is measured by means of the temperature sensor 16 of the hearing aid.

The EWS 40 will be derived by assigning to each of the measured biometric quantities 42 a corresponding criticality value c44, c46, c48, c50, c52 out of the integers from 0 to 3 in a mapping yet to be described, and taking the sum of all criticality values as the final EWS 40. However, in order to derive the EWS 40, also information on a level of consciousness 54 of the user is further required. As explained above with respect to the embodiment shown in figure 1, this level of consciousness, preferably to be given as a value in the APVU scale, may be involved to assess, such that a continuous monitoring is considered not desirable in the present embodiment.

Instead, from the biometric quantities available via the data of the PPG sensor 18 and the body temperature 52, a preliminary warning score (PWS) 60 is derived. This is done by first assigning to each of the heart rate 44, the oxygen saturation 46, the blood pressure 48, the respiratory rate 50 and the body temperature 52 its respective criticality value c44, c46, c48, c50, c52, as mentioned above, by means of mapping, e.g., according to the following array:

| Quantity\crit. val. | 3 | 2 | 1 | 0 | 1 | 2 | 3 |
|---|---|---|---|---|---|---|---|
| Resp. rate | >35 | 31-35 | 21-30 | 9-20 | | | <7 |
| O₂ saturation (%) | <85 | 85-89 | 90-92 | >92 | | | |
| Temperature (°C) | | >38.9 | 38-38.9 | 36-37.9 | 35-35.9 | 34-34.9 | <34 |
| Heart rate | >129 | 110-129 | 100-109 | 50-99 | 40-49 | 30-39 | <30 |
| Syst. pressure | | >199 | | 100-199 | 80-99 | 70-79 | <70 |

The respiratory and heart rates are to be taken in breaths/beats per minute, the systolic blood pressure is to be taken here in mmHg. The assignments of the criticality values as indicated in the table given above may be adapted to different intervals for the respective biometric quantities, in particular, in dependence of knowledge on individual conditions of the wearer (e.g., in case of known hyper- or hypotension).

The criticality values c44, c46, c48, c50, c52 are summed to yield the PWS 60, which is compared against a critical preliminary value cp60. Said critical preliminary value cp60 can be given, e.g., by one of the integers 3, 4 or 5. In case that the PWS 60 exceeds the critical preliminary value cp60, a level of consciousness 54 of the user shall be assessed, preferably in the way described above with help of figure 1. In particular, this process includes the playback of an appropriate acoustic stimulus 62 (which may possibly rise up to the user's pain threshold) by means of the output speaker 28, and monitoring a possible neural reaction of the user to the acoustic stimulus 62 via the EEG sensor 20, and a possible verbal reaction to the acoustic stimulus 62 via the electroacoustic input transducer 10.

The level of consciousness 54 is then assessed in the AVPU scale, and a criticality value c54 is assigned, preferably according to the following mapping for the criticality value c54:

| Score (crit. value) | 0 | 1 | 2 | 3 |
|---|---|---|---|---|
| Level of consciousness | A | V | P | U |

Said criticality value c54 for the user's level of consciousness 54 is summed to the PWS 60, i.e., to the sum of the criticality values c44, c46, c48, c50, c52, in order to generate the EWS 40. In case of an assessment of the level of consciousness 54 according to the GCS, the respective GCS components (eye/body movement, speaking ability) that are used, are classified and mapped according to the GCS onto a corresponding (possibly limited) GCS integer value which is then mapped via appropriate scaling onto the criticality value c54.

The EWS 40 is then compared to a first threshold 64. The first threshold may be given, e.g., by one of the integer values 5, 6 or 7. In case the EWS 40 exceeds the first threshold 64, then, via a communication link 66 such as an internet connection, an alert signal 68 containing an alert 69 is sent out. In particular, the communication link 66 may send the alert signal 68 via an LPWAN, which may be given by a commercial network system such as LoRa or Sigfox.

The alert signal 68 may be sent to a remote medical monitoring server (not shown), which may be implemented as a cloud service of a public or private, national or local health care system, the medical monitoring server being configured to automatically inform a physician or an ambulance in dependence of the value of the EWS 40. Additionally, or alternatively, the alert signal 68 may be directly transmitted to a pre-determined medical service person (not shown) of the user, such as a nursing staff (preferably the staff for the user's regular care), the user's general practitioner, or a specialist in charge of treatment of the user. A contact address such as an email address or an emergency phone number of the pre-determined medical service is preferably stored in a non-volatile memory (not shown) of the hearing device 4.

The alert 69 preferably contains all necessary identification data about the user, possibly further medical data such as known and potentially important pre-conditions (like diabetes, high blood pressure, a serious medical condition just recently overcome such as a stroke or a heart attack, etc.), as well as the EWS 40, and possibly the criticality values c44-c54 or the exact values of the biometric quantities 42.

Furthermore, upon the PWS 60 exceeding a second threshold (not shown) which may be different from the critical preliminary value cp60, the output speaker 28 of the hearing device 4 may playback an acoustic alert information (not shown) to the hearing of the user. The user this way can be informed about the fact that a certain or some of his vital function activities may have attained an unnormal (yet maybe still not critical) value, so that he can monitor his own situation more closely, skip physical activities of potential risk, of put himself in contact with a medical service person.

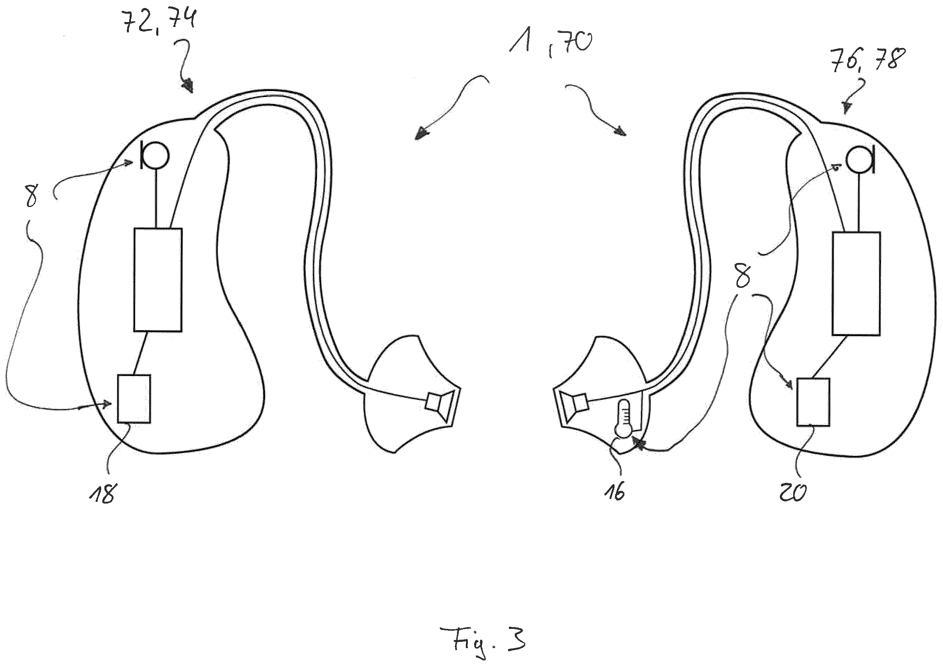

Figure 3 shows an alternative embodiment of the wearable system 1 according to figure 1. The wearable system 1 is given by a binaural hearing aid 70, comprising a left hearing aid 72 as a first ear-worn device 74 and a right hearing aid 76 as a second ear-worn device 78.

In resemblance to the hearing device 4 of figure 1 and not referenced in detail in figure 3, each of the left and right hearing aid 72, 76 comprise at least one electroacoustic input transducer for generating a respective audio input signal from an environment sound, a control unit connected to said input transducer and configured to generate an output signal by processing the audio input signal, and an output speaker for generating an output sound signal from the respective output signal.

In contrast to the hearing device 4 of figure 1, the sensors 8 of the hearing device 4 are not contained all together in either of the two hearing aids 72, 76. Instead, the PPG sensor 18 of the wearable system 1 is located in the left hearing aid 72, and the EEG sensor 20 as well as the temperature sensor 16 of the wearable system 1 are located in the right hearing aid 76. Thus, a redundant acquiring of essentially the same information by both hearing aids 72, 76 may be avoided, which allows for a more compact construction of the two hearing aids 72, 76, as well as a more balanced energy consumption due to the distribution of the sensors 8.

In order to derive the EWS, the two hearing aids 72, 76 are connected to each other via a communication link (not shown), such that the biometric quantities 42 which are measured as described according to figure 2 may be gathered in control units of one of the two hearing aids 72, 76 for a final derivation of the EWS.

Even though the invention has been illustrated and described in detail with help of a preferred embodiment example, the invention is not restricted by this example. Other variations can be derived by a person skilled in the art without leaving the extent of protection of this invention.

### reference numeral

- 1: wearable system
- 2: ear-worn device
- 4: hearing device
- 6: hearing aid
- 8: sensor
- 9: sensor signal
- 10: (electroacoustic) input transducer
- 12: environment sound
- 14: audio input signal
- 16: temperature sensor
- 18: PPG sensor
- 20: EEG sensor
- 22: EEG signal
- 24: control unit
- 26: PPG signal
- 28: output speaker
- 30: output signal
- 32: output sound signal
- 40: EWS (early warning score)
- 42: biometric quantity
- 44: heart rate
- 46: oxygen saturation
- 48: (systolic) blood pressure
- 50: respiratory rate
- 52: body temperature
- 54: level of consciousness
- 60: PWS (preliminary warning score)
- 62: acoustic stimulus
- 64: first threshold
- 66: communication link
- 68: alert signal
- 69: alert
- 70: binaural hearing aid
- 72: left hearing aid
- 74: first ear-worn device
- 76: right hearing aid
- 78: second ear-worn device
- c44, c46, c48, c50, c52, c54: criticality value
- cp60: critical preliminary value (for PWS)

## Claims

1. A wearable system (1), comprising an ear-worn device (2),
wherein the ear-worn device (2) comprises a number of sensors (8), each of which is configured to generate a respective sensor signal (9) when the wearable system (1) is worn by a user of the wearable system (1), each sensor signal (9) being representative of at least one vital function activity of said user,
wherein the wearable system (1) is configured to
- derive from said sensor signal or signals (9) at least three biomedical quantities (42) of the user out of the following: a heart rate (44), an oxygen saturation (46) of the blood, a blood pressure (48) and a respiratory rate (50), wherein to each of the at least three biometric quantities (42), a criticality value (c44, c46, c48, c50) is assigned, and
- derive an early warning score (40) from the biomedical quantities (42) and from said criticality values (c44, c46, c48, c50),
wherein a level of consciousness (54) of the user is assessed as a further biometric quantity (42), and a respective criticality value (c54) is assigned to the assessed level of consciousness (54), and
wherein the early warning score (40) is further derived from said criticality value (c54) corresponding to the level of consciousness (54).
wherein a preliminary warning score (60) is derived from criticality values (c44, c46, c48, c50, c52) corresponding to at least some biometric quantities (42) other than the level of consciousness (54),
wherein the level of consciousness (54) is assessed when the preliminary warning score (60) exceeds a critical preliminary value (cp60), and
wherein the early warning score (40) is derived based on the preliminary warning score (60) and the level of consciousness (54).

2. The wearable system (1) according to claim 1,
further comprising in said number of sensors (8) a temperature sensor (16) configured to measure a body temperature (16) of the user,
wherein the wearable system (1) is further configured to derive the early warning score (40) also based on said body temperature (16).

3. The wearable system (1) according to claim 1 or claim 2,
wherein the ear-worn device (2) comprises, in said number of sensors (8), a photoplethysmographic sensor (18) and/or an electroacoustic input transducer (10), and
wherein the wearable system (1) is configured to derive the heart rate (44) and/or the oxygen saturation (46) and/or the blood pressure (48) and/or the respiratory rate (50) from a sensor signal (9, 26) of said photoplethysmographic sensor (18), and/or to derive the respiratory rate (50) from an audio input signal (14) generated by the electroacoustic input transducer (10) from an environment sound (12).

4. The wearable system (1) according to one of claims 1 to 3,
wherein the ear-worn device (2) further comprises an electroencephalographic sensor (20) in said number of sensors (8), and
wherein the level of consciousness (54) is assessed based on a sensor signal (9, 22) of the electroencephalographic sensor (20).

5. The wearable system (1) according to one of claims 1 to 4,
comprising a first ear-worn device (74) and a second ear-worn device (78) configured to be worn by the user at different ears,
wherein the first ear-worn device (74) comprises at least first sensor (8) of said number of sensors (8), and the second ear-worn device (78) comprises at least a second sensor (8) of said number of sensors (8) measuring a different biometrical quantity (42) than the first sensor.

## Patentansprüche

1. Tragbares System (1), umfassend eine am Ohr tragbare Vorrichtung (2),
wobei die am Ohr tragbare Vorrichtung (2) eine Anzahl von Sensoren (8) umfasst, von denen jeder so konfiguriert ist, dass er ein entsprechendes Sensorsignal (9) erzeugt, wenn das tragbare System (1) von einem Benutzer des tragbaren Systems (1) getragen wird, wobei jedes Sensorsignal (9) für mindestens eine Vitalfunktionsaktivität des Benutzers repräsentativ ist,
wobei das tragbare System (1) dazu konfiguriert ist,
- von dem Sensorsignal oder den Sensorsignalen (9) mindestens drei biomedizinische Größen (42) des Benutzers von den Folgenden abzuleiten: einer Herzfrequenz (44), einer Sauerstoffsättigung (46) des Blutes, einem Blutdruck (48) und einer Atemfrequenz (50), wobei jeder der mindestens drei biometrischen Größen (42) ein Kritikalitätswert (c44, c46, c48, c50) zugeordnet wird, und
- einen Frühwarnwert (40) von den biomedizinischen Größen (42) und den Kritikalitätswerten (c44, c46, c48, c50) abzuleiten,
wobei als weitere biometrische Größe (42) ein Bewusstseinsgrad (54) des Benutzers bewertet wird und dem bewerteten Bewusstseinsgrad (54) ein entsprechender Kritikalitätswert (c54) zugeordnet wird, und
wobei der Frühwarnwert (40) weiter von dem Kritikalitätswert (c54) abgeleitet wird, der dem Bewusstseinsgrad (54) entspricht,
wobei ein vorläufiger Warnwert (60) von Kritikalitätswerten (c44, c46, c48, c50, c52) abgeleitet wird, die mindestens einigen biometrischen Größen (42), die nicht der Bewusstseinsgrad (54) sind, entsprechen,
wobei der Bewusstseinsgrad (54) bewertet wird, wenn der vorläufige Warnwert (60) einen kritischen vorläufigen Wert (cp60) überschreitet und
wobei der Frühwarnwert (40) basierend auf dem vorläufigen Warnwert (60) und dem Bewusstseinsgrad (54) abgeleitet wird.

2. Tragbares System (1) nach Anspruch 1,
wobei die Anzahl der Sensoren (8) weiter einen Temperatursensor (16) umfasst, der dazu konfiguriert ist, eine Körpertemperatur (16) des Benutzers zu messen,
wobei das tragbare System (1) weiter dazu konfiguriert ist, den Frühwarnwert (40) auch basierend auf der Körpertemperatur (16) abzuleiten.

3. Tragbares System (1) nach Anspruch 1 oder Anspruch 2,
wobei die am Ohr getragene Vorrichtung (2) in der Anzahl von Sensoren (8) einen photoplethysmographischen Sensor (18) und/oder einen elektroakustischen Eingangswandler (10) umfasst und
wobei das tragbare System (1) dazu konfiguriert ist, die Herzfrequenz (44) und/oder die Sauerstoffsättigung (46) und/oder den Blutdruck (48) und/oder die Atemfrequenz (50) von einem Sensorsignal (9, 26) des photoplethysmographischen Sensors (18) abzuleiten und/oder die Atemfrequenz (50) von einem Audio-Eingangssignal (14) abzuleiten, das von dem elektroakustischen Eingangswandler (10) aus einem Umgebungsgeräusch (12) erzeugt wird.

4. Tragbares System (1) nach einem der Ansprüche 1 bis 3,
wobei die am Ohr getragene Vorrichtung (2) weiter einen elektroenzephalografischen Sensor (20) in der Anzahl von Sensoren (8) umfasst und
wobei der Bewusstseinsgrad (54) basierend auf einem Sensorsignal (9, 22) des elektroenzephalografischen Sensors (20) bewertet wird.

5. Tragbares System (1) nach einem der Ansprüche 1 bis 4,
umfassend eine erste am Ohr getragene Vorrichtung (74) und eine zweite am Ohr getragene Vorrichtung (78), die dazu konfiguriert sind, dass sie vom Benutzer an unterschiedlichen Ohren getragen werden können,
wobei die erste am Ohr getragene Vorrichtung (74) mindestens einen ersten Sensor (8) der Anzahl von Sensoren (8) umfasst und die zweite am Ohr getragene Vorrichtung (78) mindestens einen zweiten Sensor (8) der Anzahl von Sensoren (8) umfasst, der eine andere biometrische Größe (42) misst als der erste Sensor.

## Revendications

1. Système portable (1), comprenant un dispositif porté sur l'oreille (2),
dans lequel le dispositif porté sur l'oreille (2) comprend un certain nombre de capteurs (8), chacun étant configuré pour générer un signal de capteur respectif (9) lorsque le système portable (1) est porté par un utilisateur du système portable (1), chaque signal de capteur (9) étant représentatif d'au moins une activité de fonction vitale dudit utilisateur,
dans lequel le système portable (1) est configuré pour
- déduire dudit signal ou desdits signaux de capteur (9) au moins trois grandeurs biomédicales (42) de l'utilisateur parmi les suivantes : une fréquence cardiaque (44), une saturation en oxygène (46) du sang, une pression artérielle (48) et une fréquence respiratoire (50), dans lequel une valeur de criticité (c44, c46, c48, c50) est attribuée à chacune des au moins trois grandeurs biométriques (42), et
- déduire un score d'alerte précoce (40) à partir des grandeurs biomédicales (42) et desdites valeurs de criticité (c44, c46, c48, c50),
dans lequel un niveau de conscience (54) de l'utilisateur est évalué comme étant une grandeur biométrique (42) supplémentaire, et une valeur de criticité respective (c54) est attribuée au niveau de conscience évalué (54), et
dans lequel le score d'alerte précoce (40) est en outre déduit de ladite valeur de criticité (c54) correspondant au niveau de conscience (54),
dans lequel un score d'avertissement préliminaire (60) est déduit de valeurs de criticité (c44, c46, c48, c50, c52) correspondant à au moins certaines grandeurs biométriques (42) autres que le niveau de conscience (54),
dans lequel le niveau de conscience (54) est évalué lorsque le score d'avertissement préliminaire (60) dépasse une valeur préliminaire critique (cp60), et
dans lequel le score d'alerte précoce (40) est déduit sur la base du score d'alerte préliminaire (60) et du niveau de conscience (54).

2. Système portable (1) selon la revendication 1,
comprenant en outre dans ledit nombre de capteurs (8) un capteur de température (16) configuré pour mesurer une température corporelle (16) de l'utilisateur,
dans lequel le système portable (1) est en outre configuré pour déduire le score d'alerte précoce (40) également sur la base de ladite température corporelle (16).

3. Système portable (1) selon la revendication 1 ou la revendication 2,
dans lequel le dispositif porté sur l'oreille (2) comprend, dans ledit nombre de capteurs (8), un capteur photopléthysmographique (18) et/ou un transducteur d'entrée électroacoustique (10), et
dans lequel le système portable (1) est configuré pour déduire la fréquence cardiaque (44) et/ou la saturation en oxygène (46) et/ou la pression artérielle (48) et/ou la fréquence respiratoire (50) à partir d'un signal de capteur (9, 26) dudit capteur photopléthysmographique (18), et/ou pour déduire la fréquence respiratoire (50) à partir d'un signal d'entrée audio (14) généré par le transducteur d'entrée électroacoustique (10) à partir d'un son ambiant (12).

4. Système portable (1) selon l'une des revendications 1 à 3,
dans lequel le dispositif porté sur l'oreille (2) comprend en outre un capteur électroencéphalographique (20) dans ledit nombre de capteurs (8), et
dans lequel le niveau de conscience (54) est évalué sur la base d'un signal de capteur (9, 22) du capteur électroencéphalographique (20).

5. Système portable (1) selon l'une des revendications 1 à 4,
comprenant un premier dispositif porté sur l'oreille (74) et un second dispositif porté sur l'oreille (78) configurés pour être portés par l'utilisateur sur des oreilles différentes,
dans lequel le premier dispositif porté sur l'oreille (74) comprend au moins un premier capteur (8) dudit nombre de capteurs (8) et le second dispositif porté sur l'oreille (78) comprend au moins un second capteur (8) dudit nombre de capteurs (8) mesurant une grandeur biométrique différente (42) de celle du premier capteur.
